# EUROPEAN PATENT APPLICATION

(11) **EP 2 629 096 A1**
(43) Date of publication of application: **21.08.2013**
(21) Application number: 12156192.2
(22) Date of filing: 20.02.2012
(51) Int. Cl.: G01N 33/576, G01N 33/68

(54) **HBV immunocomplexes for response prediction and therapy monitoring of chronic HBV patients**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention relates to a method for identifying a subject suffering from hepatitis B virus (HBV) infection as being susceptible to interferon treatment, said method comprising the steps of a) determining, in a sample of said subject, the amount of HBV immune complexes, b) comparing the amount of HBV immune complexes obtained in step a) to a reference value, and c) identifying a subject suffering from HBV infection as being susceptible to interferon treatment based on the result of the comparison made in step b). The present invention further relates to the use of the determination of the amount of HBV immune complexes in a sample from a subject suffering from HBV infection and of a detection agent for HBV immune complexes for identifying a subject suffering from HBV infection as being susceptible to interferon treatment. Furthermore, the present invention relates to a device and a kit allowing identifying a subject suffering from HBV infection as being susceptible to interferon treatment.

## Description

The present invention relates to the field of diagnostics. In particular, it relates to a method for identifying a subject suffering from hepatitis B virus (HBV) infection as being susceptible to interferon treatment, said method comprising the steps of a) determining, in a sample of said subject, the amount of HBV immune complexes, b) comparing the amount of HBV immune complexes obtained in step a) to a reference value, and c) identifying a subject suffering from HBV infection as being susceptible to interferon treatment based on the result of the comparison made in step b). The present invention further relates to the use of the determination of the amount of HBV immune complexes in a sample from a subject suffering from HBV infection and of a detection agent for HBV immune complexes for identifying a subject suffering from HBV infection as being susceptible to interferon treatment. Furthermore, the present invention relates to a device and a kit allowing identifying a subject suffering from HBV infection as being susceptible to interferon treatment.

Hepatitis B is an infectious inflammatory disease of the liver caused by the hepatitis B virus (HBV). The virus is transmitted by exposure to body fluids, transmission by transfusions, dialysis, common use of needles by drug-addicted persons, and perinatal infection being the major modes of transmission.

Acute Hepatitis B virus infection is characterized by liver inflammation, vomiting, and jaundice. Symptoms typically last for a few weeks and then gradually improve while the immune system clears the infection. The probability of full recovery and establishment of protective immunity increases with age: While only 5% adults will suffer from a persistent infection lasting for more than a few weeks, i.e. chronic HBV infection, this rate rises to 70% for young children, and to 95% for newborns infected perinatally.

Chronic HBV is associated with a severe risk of developing cirrhosis and liver cancer, which is why HBV has been classified as a class I carcinogen, i.e. an agent carcinogenic to humans, by WHO's International Agency for the Research on Cancer (IARC). It is thus of outmost importance for the patient to receive a treatment allowing the patient's immune system to clear the infection. Typical treatment schemes include nucleoside or nucleotide analogs inhibiting the viral DNA polymerase, along with Interferon alpha. An important improvement was the introduction of PEGylated interferon (e.g. Pegasys®), providing prolonged half-life of interferon, into therapy. For review of epidemiology and therapy of HBV infection see J.L. Dienstag N Engl J Med 2008, 359; 1486-1500.

The serology of chronic HBV infection is complex, since on the one hand high anti-HBV titers are directly correlated with the severity of disease, but on the other hand anti-HBV e antigen antibodies were found to be present in patients years before spontaneous seroconversion, i.e. clearance of the HBV e antigen from serum (Maruyama et al. (1993), J. Clin. Invest. 91, 2586-2595). Despite improved therapy, still only a portion of patients will clear the infection under treatment, while others will not. Members of the second group of patients, the non-responders, can frequently clear the infection under a more aggressive therapy regimen, like e.g. increased doses, prolonged treatment, or additional medication. It would, thus, be desirable to be able to predict a patient's response to standard interferon therapy, in order to possibly adjust treatment right from the beginning. In a recent improvement, it was found that determination of a change in HBV soluble antigen (HBsAg) in patient samples after 12 weeks of therapy allows the use of cutoff values for the prediction of therapy outcome. However, cutoff values are only valid for patients being also HBV e antigen (HBeAg) positive, which is the case mainly in patients in the Asian-Pacific area. In HBeAg negative patients, which are the majority e.g. in Europe, cutoff values are much more difficult to define and a decrease of at least 10% between therapy start and week 12 is used as a makeshift.

Recently, it was reported that in HBeAg positive patients, a high amount of HBV complexes comprising HBsAG and anti-HBsAg-IgG before the start of therapy was correlated with a good response to standard interferon therapy. However, this does not allow prediction for HBeAg negative patients. At any rate, at present no method is available that would allow to predict therapy outcome before the therapy is started for all patient groups regardless of their HBeAg status.

Accordingly, the technical problem underlying the present invention can be seen as the provision of means and methods for complying with the aforementioned needs. The technical problem is solved by the embodiments characterized in the claims and herein below.

Therefore, the present invention relates to a method for identifying a subj ect suffering from hepatitis B virus (HBV) infection as being susceptible to interferon treatment, said method comprising the steps of
a) determining, in a sample of said subject, the amount of HBV immune complexes,
b) comparing the amount of HBV immune complexes obtained in step a) to a reference value; and
c) identifying a subject suffering from HBV infection as being susceptible to interferon treatment based on the result of the comparison made in step b).

The method of the present invention, preferably, is an in vitro method. Moreover, it may comprise steps in addition to those explicitly mentioned above. For example, further steps may relate, e.g., to pre-treatment of the sample of step a) or evaluation of the results obtained by the method. Additionally, internal controls, such as sample quality controls or performance controls may be used. The method may be carried out manually or assisted by automation. Preferably, steps (a) to (c) may in total or in part be assisted by automation, e.g. by suitable robotic and sensory equipment for the determining of HBV immune complexes in step (a).

The term "identifying", as used herein, means to allocate a subject into the group of subjects being susceptible to interferon treatment (so-called "responder") or into the group of subjects not being susceptible to interferon treatment (so-called "non-responders"). As will be understood by those skilled in the art, the aforementioned identification is usually not intended to be correct for 100% of the subjects to be analyzed. The term, however, requires that the assessment will be valid for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the differentiation will be correct for at least 60%, at least 70%, at least 80%, or at least 90% of the subjects of a given cohort or population.

Preferably, identifying according to the method of the invention can be accomplished in the absence of knowledge about the HBV e antigen status of the subject, i.e. the identifying leads to a correct allocation to the group of subjects being susceptible to interferon treatment or to the group of subjects not being susceptible to interferon treatment for subjects regardless of the HBeAg status of the investigated subject. Thus, the method can be applied for subjects with detectable amounts of HBeAg in serum samples as well as for subjects lacking detectable amounts of HBeAg in serum samples.

The term "hepatitis B virus (HBV)" refers to a virus species from the family hepadnaviridae which is the causative agent of hepatitis B. HBV virus particles consist of an outer membrane (also called lipid envelope), an icosahedral nucleocapsid and DNA genome. HBV is well known and characterized in the art.

The terms "hepatitis B virus infection" or "HBV infection" relate to the detectable presence of HBV in a subject. Preferably, HBV presence is diagnosed by the detection of at least one viral polypeptide in a sample from a subject, more preferably, at least one of the viral antigens HBs (HBsAg, Genbank Acc. No.: AAL66340.1 GI:18252577, SEQ ID NO: 1), HBc (HBcAg, Genbank Acc. No.: CAA51257.1 GI:288930, SEQ ID NO: 2), and HBe (HBeAg, Genbank Acc. No.: AAM96930.1 GI:22530876, SEQ ID NO: 3) is detected. It is understood by the skilled person that the HBV polypeptides are referenced as biomarkers, not as specific polypeptides, and that the term HBV encompasses various strains of HBV which may comprise sequence variants of the aforementioned HBV polypeptides. Accordingly, the aforementioned polypeptides having the specific sequences deposited under the Genbank accession numbers are to be understood as exemplary sequences representing a biomarker. Encompassed according to the present invention are also variant polypeptides which vary due to at least one amino acid addition, substitution and/or deletion form the polypeptide having the specific sequence as long as they are also suitable as biomarkers for a HBV infection as discussed above. Preferably, the variant polypeptides are at least 70%, at least 75%, at least 80%, at least 85%, at least 90%, at least 95%, at least 98% or at least 99% identical to the specific polypeptides. The term "identical" as used herein refers to sequence identity characterized by determining the number of identical amino acids between two nucleic acid sequences or amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

Also preferably, HBV presence is detected by detecting at least one viral polynucleotide, preferably viral DNA, in a sample from a subject. The nucleotide sequences of the viral polynucleotides or the entire HBV genome are well known in the art. Preferably, HBV nucleotide sequences are deposited under Genbank accession numbers NC_003977.1. It is understood by the skilled person that the HBV polynucleotides are referenced as biomarkers, not as specific polynucleotides, and that the term HBV encompasses various strains of HBV comprising variant nucleotide sequences. Accordingly, the aforementioned polynucleotides having the specific sequences deposited under the Genbank accession numbers are to be understood as exemplary sequences representing a biomarker. Encompassed according to the present invention are also variant polynucleotides which vary due to at least one nucleotide addition, substitution and/or deletion form the polynucleotides having the specific sequence as long as they are also suitable as biomarkers for a HBV infection as discussed above. Preferably, the variant polynucleotides peptides are at least 70%, at least 75%, at least 80%, at least 85%, at least 90%, at least 95%, at least 98% or at least 99% identical to the specific polynucleotides. Identity can be calculated as set forth elsewhere herein for amino acid sequences.

Preferably, the HBV infection referred to herein is a chronic HBV infection, wherein said chronic HBV infection is, preferably, characterized by the detectable presence of HBV in a subject for more than four weeks, more than five weeks, more than six weeks, more than seven weeks, more than eight weeks, more than nine weeks, more than ten weeks, more than eleven weeks, or more than twelve weeks. More preferably, a chronic HBV infection referred to herein follows the definition published by the Center for Disease Control (CDC), according to which a chronic HBV infection is characterized by the following laboratory criteria: IgM antibodies to hepatitis B core antigen (IgM anti-HBc) negative AND a positive result on one of the following tests: hepatitis B surface antigen (HBsAg), hepatitis B e antigen (HBeAg), or hepatitis B virus (HBV) DNA OR HBsAg positive or HBV DNA positive two times at least 6 months apart (any combination of these tests performed 6 months apart is acceptable).

The terms "interferon treatment" and "standard treatment", preferably, relate to a treatment of HBV infection with interferon and, preferably, an inhibitor of the viral DNA polymerase. It is, however, also contemplated by the present invention that interferon treatment preferably is single treatment with interferon and that standard treatment preferably is single treatment with a viral DNA polymerase inhibitor. Preferably, interferon as referred to in this context is an interferon covalently bound to polyethylenglycol (PEG-interferon), more preferably, interferon is interferon 2 alpha, most preferably, interferon is interferon 2 alpha covalently bound to polyethylenglycol (PEG-interferon 2 alpha, commercially available as PEGasys®). Preferably, an inhibitor of the viral DNA polymerase is a nucleotide or nucleoside analogon, more preferably {[2-(6-amino-9H-purin-9-yl)ethoxy]methyl}phosphonic acid (e.g. adefovir®). Thus, standard or interferon treatment, preferably, is treatment with interferon 2 alpha and a nucleotide analogon. More preferably, standard or interferon treatment is treatment with PEG-interferon 2 alpha and a nucleotide analogon and even more preferably standard interferon treatment is treatment with PEG-interferon 2 alpha (e.g. PEGasys®) and adefovir® for less than one year. Most preferably, standard interferon treatment is treatment with PEG-interferon 2 alpha, e.g. PEGasys®, and adefovir® according to the following therapy plan: Usually, the PEGasys® treatment is carried out by subcutaneous administration (weekly subcutaneous injection) for a duration of 48 weeks whereas the viral DNA polymerase inhibitors (nucleoside/nucleotide analoga) are administrated orally for a longer time, i.e. for more than one year in 80% of the patients. For details see also J.L. Dienstag N Engl J Med 2008, 359; 1486-1500.

The term "being susceptible to interferon treatment" means that a subject can be successfully treated or can at least be successfully treated with a significantly increased likelihood compared to the prevalence for a successful treatment. Subjects which can be successfully treated by interferon treatment as referred to herein are also called responders. Treatment as referred to herein is successful if the HBV infection, at least one symptom associated therewith or at least one complication accompanied therewith are ameliorated to a significant extent and/or cured. Preferably, a successful treatment is also accompanied by a decrease of HBV polypeptide and /or HBV polynucleotide detectable in a sample from a subject as described herein above. More preferably, successful treatment is characterized by the absence of detectable amounts of HBsAG and / or HBeAG in samples from a subject after at most one year, at most 50 weeks, at most 49 weeks, or at most 48 weeks of treatment as determined 60 to 80 weeks, preferably 65 to 75 weeks, or most preferably 72 weeks after start of the treatment.

A subject which can not successfully treated by interferon treatment is also called non-responder. In such subjects, no amelioration or cure of the HBV infection, the at least one symptom or at least one complication associated therewith occurs. Preferably, such a non-responder subject suffers from a severe form of chronic hepatitis B virus infection wherein HBsAG, but not HBeAG, is detectable before and after interferon treatment, or wherein HBsAG and HBeAG are detectable before and after standard interferon treatment. A subject, identified as being not susceptible to interferon treatment shall be regarded for modified treatment measures, e.g. increased doses of interferon and/or polymerase inhibitor, and/or prolonged treatment and/or additional medication.

As used herein, the term "HBV immune complex" relates to a complex formed between at least one HBV polypeptide and at least one immunoglobulin as described herein below directed against said at least one HBV polypeptide, i.e. an anti-HBV immunoglobulin. Preferably, the complex is formed between at least one HBV polypeptide and more than one, more than two, more than three, more than four, more than five, more than ten, more than twenty, more than fifty, or more than a hundred anti-HBV immunoglobulin molecules directed against said at least one HBV polypeptide. Preferably, the HBV polypeptide is elected from the list of HBV antigens related to herein above, more preferably, the HBV polypeptide is the HBsAG. The anti-HBV immunoglobulin, preferably, is a soluble immunoglobulin present in at least one of a subject's body fluids, preferably blood or serum. Preferably, the anti-HBV immunoglobulin is an IgG, more preferably a serum IgG. Most preferably, the anti-HBV immunoglobulin comprised in the HBV immune complex is an IgG directed against the HBsAg, i.e. an anti-HBsAg IgG. It is to be understood that the HBV immune complex may comprise other molecules than the ones expressly detailed in this specification, it is, however, preferred that the anti-HBV immunoglobulin and the HBV polypeptide of the present invention form a substantial part of the HBV immune complex. Thus, the anti-HBV immunoglobulin and the HBV polypeptide constitute, preferably, at least 25%, at least 30%, at least 40%, at least 50%, at least 60%, at least 70%, or at least 75% of the mass of the HBV immune complex. More preferably, HBV immune complex essentially consists of the anti-HBV immunoglobulin and the HBV polypeptide, i.e. the anti-HBV immunoglobulin and the HBV polypeptide constitute at least 80%, at least 90%, or at least 95% of the mass of the HBV immune complex. Even more preferably, HBsAg and anti-HBsAg IgG constitute a substantial part of the HBV immune complex. Most preferably, the HBV immune complex essentially consists of HBsAg and anti-HBsAg IgG.

The terms "immunoglobulin" or "Ig" are understood by the skilled person. They relate to a member molecule of the family of immunoglobulins, characterized in that they are soluble polypeptides produced by the immune system of an individual in response to contact with a foreign antigen. Preferably, the immunoglobulin is an immunoglobulin from a mammal and, more preferably, the immunoglobulin is a human immunoglobulin. Also preferably, the immunoglobulin is elected from the group consisting of IgA, IgD, IgE, IgG, and IgM. More preferably, the immunoglobulin is an IgG, most preferably a human IgG. Human IgGs are characterized by a molecular mass of approximately 150 kDa and by a structure consisting of two identical heavy chains of about 50 kDa and two identical light chains of approximately 25 kDa. The two heavy chains are linked to each other and to a light chain by disulfide bonds to form the typical Y-shaped molecule. Preferably, the IgG are glycosylated, more preferably IgG are glycosylated by N-glycosylation. Preferably, the IgG is of one of the subgroups IgG1, IgG2, IgG3, or IgG4.

The term "anti-immunoglobulin antibody" relates, preferably, to a soluble molecule from the protein family of antibodies recognizing an immunoglobulin, preferably recognizing a human immunoglobulin, i.e. the anti-immunoglobulin antibody of the present invention preferably is an anti-human-immunoglobulin antibody, more preferably recognizing a human IgG, i.e. an anti-human-IgG antibody. The anti-immunoglobulin antibody according to the present invention is preferably an antibody with low to medium affinity and with high avidity as detailed herein below. The affinity of an antibody for an epitope is defined as the strength of all non-covalent interactions between the antigen-recognition site on said antibody and the epitope. An antibody with a high affinity binds strongly to an antigen via many and / or strong non-covalent interactions and, thus, remains bound to the antigen for a relatively long period of time. An antibody with a low affinity, on the other hand, interacts with few and / or weak non-covalent interactions and thus dissociates rapidly from the antigen. It is known to the person skilled in the art that the affinity of an antibody can be described by it dissociation constant (K_{d}). Preferably, a dissociation constant of 10⁻¹⁰ mol/l to 10⁻⁹ mol/l is indicative of a very high affinity, a dissociation constant of 10⁻⁸ mol/l is indicative of a high affinity, a dissociation constant of 10⁻⁷ mol/l is indicative of a low affinity, and a dissociation constant of 10⁻⁶ mol/l and higher is indicative of a very low affinity. Thus, the dissociation constant of the anti-immunoglobulin antibody, preferably, is 10⁻⁶ mol/l to 10⁻⁸ mol/l, more preferably the dissociation constant is 10⁻⁷ to 10⁻⁸ mol/1. In molecules comprising more than one binding site, like e.g. antibodies, the interaction of a first binding site increases the probability of a further binding site to interact. The strength of such multiple interactions between a molecule comprising more than one binding site and a molecule to be bound is known to the skilled artisan as avidity. A high avidity can thus compensate for a relatively low affinity of the single binding site. Thus, the anti-immunoglobulin antibody, preferably, has more than two antigen recognition sites, more preferably more than four antigen recognition sites. Most preferably, the anti-immunoglobulin antibody has ten or more antigen recognition sites. Preferably, the anti-immunoglobulin antibody is an IgG, IgD, IgE, more preferably the anti-immunoglobulin antibody is an antibody comprising more than two antigen recognition sites, e.g. an IgA, most preferably, the anti-immunoglobulin antibody is an antibody comprising more than four antigen recognition sites in one molecule, e.g. an IgM. The anti-immunoglobulin antibody is polyclonal or monoclonal and is produced in a mammal or in a mammalian cell suited for antibody production. Preferably, the anti-immunoglobulin antibody specifically binds to an epitope of an immunoglobulin of the present invention. More preferably, the anti-immunoglobulin antibody is an anti-human-immunoglobulin antibody, i.e. binds to the human immunoglobulin of the present invention, even more preferably, the binding is specific for human immunoglobulin. Most preferably, the anti-immunoglobulin antibody is an anti-human-IgG antibody, i.e. specifically binds to human IgG. It is, however, also envisaged by the present invention that the anti-immunoglobulin antibody specifically binds to a neoepitope formed by the binding of an immunoglobulin to the HBV polypeptide in the HBV immune complex. Methods of how to produce antibodies are well known in the art, and include, e.g., immunization of live animals according to known protocols or the production of monoclonal or polyclonal antibodies in cell culture systems (Sambrook et al., 1989, Molecular Cloning: A Laboratory Manual). Preferably, the anti-immunoglobulin antibody is produced in mouse, rat, rabbit, pig, cow, donkey, goat, sheep, or the like or in eggs. It is, however, also considered by the present invention that the anti-immunoglobulin antibody is produced in a transgenic plant. Preferably, the anti-immunoglobulin antibody is an IgM, most preferably a monoclonal IgM. More preferably, the anti-immunoglobulin antibody is a anti-(aggregated-humanIgG) IgM, i.e. an IgM with a low affinity and a high avidity for human IgG as described herein above, e.g. the <h-agg.IgG> IgM as described in WO2008/135274, most preferably, the anti-human-immunoglobulin antibody is. MAb <h-Agg.-IgG>M-3.022.5-IgM (DSM ACC2873), MAb <h-Agg.-IgG>M-1.010.2-IgM and MAb <h-Agg.-IgG>M-1.1.7-IgM (shown in Table 1), MAb <h-Agg.-IgG>M-3.022.5-IgM (DSM ACC2873) being most preferred.

Preferably, the anti-immunoglobulin antibody carries a label. The term "label" as used herein relates to any substance capable of producing a detectable signal. Preferably, the label is a chromogen, a fluorescent, chemiluminescent or electrochemiluminscent compound, a catalyst, an enzyme, a an enzymatic substrate, a dye, a colloidal metal or nonmetallic particle, or an organic polymer particle, or the like.

The term "subject", as used herein, relates to a mammal and, preferably, to a human. The subject, preferably, suffers from HBV infection. More preferably, the subject has a chronic HBV infection. Moreover, the subject has, preferably, an unknown HBV e antigene status.

The term "sample" refers to a sample of a body fluid, to a sample from a tissue or an organ or to a sample of wash/rinse fluid obtained from an outer or inner body surface. The sample preferably comprises polypeptides, more preferably HBV antigens, most preferably HBsAg. Samples of blood, plasma, serum, urine, saliva, or lacrimal fluid are encompassed by the method of the present invention. Such samples can be obtained by use of brushes, (cotton) swabs, spatula, rinse/wash fluids, punch biopsy devices, puncture of cavities with needles or surgical instrumentation. However, samples obtained by well known techniques including, preferably, scrapes, swabs or biopsies from the urogenital tract, perianal regions, anal canal, the oral cavity, the upper aerodigestive tract and the epidermis are also included as samples of the present invention. Cell-free fluids may be obtained from the body fluids or the tissues or organs by separating techniques such as filtration or centrifugation. Preferably, samples are obtained from body fluids known to comprise HBV polypeptides in subjects infected with HBV, i.e., preferably, blood, serum, saliva, or the like. It is to be understood that the sample may be further processed in order to carry out the method of the present invention. Particularly, cells might be removed from the obtained sample by methods and means known in the art. Moreover, HBV immune complexes might be extracted and/or purified from the obtained sample by methods and means known in the art. Thus, the term sample also may relate to HBV immune complexes purified and/or extracted from any sample as mentioned above.

The term "determining" relates to the quantification of the amount of HBV immune complex present in a sample, i.e. measuring the amount or concentration of said HBV immune complex, preferably semi-quantitatively or quantitatively. Measuring can be done directly or indirectly. The determining of the amount of immune complexes can be accomplished in a variety of ways known to the skilled person, e.g. gel filtration chromatography followed by western blotting, co-immunoprecipitation, or the like. Preferably, the amount of HBV immune complexes is determined by the methods described in the examples herein below. Advantageously, it was found that the sandwich-ELISA used in the examples allows reliable quantification of HBV immune complexes. Moreover, it was found that the use of aggregated antibodies, e.g. aggregated IgG, as anti-human-Ig antibodies, leads to a highly improved signal-to-noise ratio.

In accordance with the present invention, determining the amount of the HBV immune complexes can be achieved by all known means for determining the amount of a polypeptide or peptide in a sample, provided that they are adapted to specifically detect the HBV immune complexes of the present invention. Preferably, detection agents are to be used which specifically bind to and, thus, allow for the detection of the HBV immune complexes. Detection agents, preferably, encompass antibodies or fragments thereof that specifically bind to the complexes, aptameres, anticalins, or Designed Ankyrin Repeat Proteins (DARPins) that specifically bind to the complexes. Preferably, double-specificity immunoassays are applied, i.e. assays wherein the presence or the intensity of a signal will depend on the presence of both kinds of molecules comprised in the HBV immune complexes, i.e. the HBV polypeptide and the anti-HBV immunoglobulin. Said means comprise immunoassay devices and methods which may utilize labeled molecules in various sandwich, competition, or other assay formats. Said assays will develop a signal which is indicative of the presence or absence of the HBV immune complexes. Moreover, the signal strength can, preferably, be correlated directly or indirectly (e.g. reverse-proportional) to the amount of HBV immune complexes present in a sample. Said methods comprise, preferably, biosensors, optical devices coupled to immunoassays, biochips, analytical devices such as mass-spectrometers, NMR-analyzers, or chromatography devices. Further, methods include micro-plate ELISA-based methods, fully-automated or robotic immunoassays (available for example on multi parameter biochip platforms or ElecsysTM analyzers), CBA (an enzymatic Cobalt Binding Assay, available for example on Roche-HitachiTM analyzers), and latex agglutination assays (available for example on Roche-HitachiTM analyzers).

The term "amount" as used herein encompasses the absolute amount of the HBV immune complexes referred to herein, the relative amount or concentration of the HBV immune complexes referred to herein as well as any value or parameter which correlates thereto. Such values or parameters comprise intensity signal values from all specific physical or chemical properties obtained from the HBV immune complexes referred to herein by measurements, e.g., expression levels determined from biological read out systems in response to the polypeptides referred to herein or intensity signals obtained from specifically bound ligands. It is to be understood that values correlating to the aforementioned amounts or parameters can also be obtained by all standard mathematical operations.

"Comparing" as used herein encompasses comparing the amount of the HBV immune complexes referred to herein which are comprised by the sample to be analyzed with an amount of the said HBV immune complexes in a suitable reference sample as specified elsewhere herein in this description. Also encompassed is comparing the ratio of the amount of the HBV immune complexes to the amount of HBV antigen, preferably HBsAg, in the sample to a suitable reference ratio. It is to be understood that comparing as used herein refers to a comparison of corresponding parameters or values, e.g., an absolute amount of the HBV immune complexes as referred to herein is compared to an absolute reference amount of said HBV immune complexes; a concentration of the HBV immune complexes as referred to herein is compared to a reference concentration of said HBV immune complexes; an intensity signal obtained from the HBV immune complexes as referred to herein in a test sample is compared to the same type of intensity signal of said HBV immune complexes in a reference sample; or a ratio of the amount of the HBV immune complexes to the amount of HBV antigen as referred to herein is compared to a corresponding reference ratio. The comparison referred to in the methods of the present invention may be carried out manually or computer assisted. For a computer assisted comparison, the value of the determined amount or ratio may be compared to values corresponding to suitable references which are stored in a database by a computer program.

The computer program may further evaluate the result of the comparison by means of an expert system. Accordingly, the result of the identification referred to herein may be automatically provided in a suitable output format.

The term "reference value" as used herein refers to an amount of HBV immune complexes, which allows assessing if being susceptible to interferon treatment or not being susceptible to interferon treatment is to be assumed for the subject from which the sample is derived. A suitable reference value may be determined from a reference sample to be analyzed together, i.e. simultaneously or subsequently, with the sample.

Reference amounts can, in principle, be calculated for a group or cohort of subjects as specified herein based on the average or mean values for a given HBV immune complex by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the γ-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the methods of the present invention can be generated, preferably, by establishing a ROC for said cohort as described above and deriving a threshold amount there from. Dependent on a desired sensitivity and specificity for a diagnostic method, the ROC plot allows deriving suitable thresholds.

Preferably, the reference amount as used herein is derived from samples of subjects obtained before treatment, but for which it is known if their donors responded to treatment or not. This reference amount level may be a discrete figure or may be a range of figures. Evidently, the reference level or amount may vary between individual species of HBV immune complexes. The measuring system therefore, preferably, is calibrated with a sample or with a series of samples comprising known amounts of HBV immune complex or HBV immune complexes. More preferably, the system is calibrated with a series of mixtures comprising defined volumes of HBsAg-only serum, i.e. serum comprising high amounts of HBsAg, but no anti-HBV immunoglobulin or HBV immune complexes, and anti-HBsAg immunoglobulin-only serum, e.g. serum comprising high titers of anti-HBsAg IgG, but no HBsAg or HBV immune complexes. It is understood by the skilled person that in such case the amount of HBV immune complex will preferably be expressed as arbitrary units (AU). Thus, preferably, the amounts of HBV immune complex or HBV immune complexes is or are determined by comparing the signal obtained from the sample to signals comprised in a calibration curve.

The reference amount applicable for an individual subject may vary depending on various physiological parameters such as age, gender, or subpopulation. Thus, a suitable reference amount may be determined by the methods of the present invention from a reference sample to be analyzed together, i.e. simultaneously or subsequently, with the test sample. Moreover, a threshold amount can be preferably used as a reference amount. Preferably, an amount of HBV immune complexes which is above the threshold amount is indicative of a mild form ofHBV infection; and an amount of HBV immune complexes which is equal or below the threshold amount will be indicative for a severe form of HBV infection.. It is to be understood that the aforementioned amounts may vary due to statistics and errors of measurement.

In one embodiment of the method of the present invention, it has been found that an increased amount of HBV immune complexes is, preferably, indicative of a subject being susceptible to interferon treatment while a decreased amount for HBV immune complexes is indicative for a subject being not susceptible to interferon treatment. In this case, the reference amounts are, preferably, those which are the average or mean amounts found in a subjects suffering from HBV prior to the treatment for a given population or cohort of subjects. A decrease or an increase of the HBV immune complex amounts referred to herein is, preferably, a statistically significant decrease or increase.

A reference amount may, preferably, be derived from a sample of a subject or group of subjects suffering from HBV which is/are known to be susceptible to interferon treatment. In such a case, a determined amount of the HBV immune complexes which is essentially identical or increased compared to the reference amount shall be indicative for a subject being susceptible to interferon treatment. An amount which is decreased shall be indicative for subject which is not susceptible for interferon treatment.

A reference amount may, preferably, also be derived from a sample of a subject or group of subjects suffering from HBV which is/are known not to be susceptible to interferon treatment. In such a case, a determined amount of the HBV immune complexes which is essentially identical or decreased compared to the reference amount shall be indicative for a subject not being susceptible to interferon treatment. An amount which is increased shall be indicative for subject which is not susceptible for interferon treatment.

Advantageously, it was found that the determining the amount of HBV immune complexes present in a sample from a subject allows for identifying a subject suffering from hepatitis B virus (HBV) infection as being susceptible to standard interferon treatment. As is detailed herein in the examples, preferably, a increased amount of HBV immune complexes is indicative of a subject being susceptible to interferon treatment, and a decreased amount of HBV immune complexes is indicative of a subject not being susceptible to interferon treatment. Meaning, preferably, that a subject with a high amount of HBV immune complexes has a high probability to respond to standard interferon treatment, and that a subject with a low amount of HBV immune complexes has a low probability to respond to standard interferon treatment. It is thus possible, using the method of the present invention, to decide before a therapy is started, if a subj ect should be treated with standard interferon therapy or if the subject should preferably receive a modified treatment. Also advantageously, it was found that the knowledge of the HBeAg status and, thus, the determination of the HBeAg status of a subject is irrelevant for the method of the present invention in order to correctly identify a subject susceptible to interferon treatment.

The definitions made above apply *mutatis mutandis* to the following:
The present invention also contemplates a method for treating a subject suffering from HBV infection by an interferon treatment comprising identifying the subject as being susceptible for the interferon treatment, preferably, by the aforementioned method of the invention, and administering to a subject identified as being susceptible for the interferon treatment a therapeutically effective amount of the interferon treatment specified elsewhere herein.

The present invention also relates to a method for differentiating in a subject suffering from HBV infection between a mild and a severe form of HBV infection, said method comprising the steps of:
a) determining, in a sample of said subject, the amount of HBV immune complexes,
b) comparing the amount of HBV immune complexes obtained in step a) to a reference value; and
c) differentiating between mild and severe HBV infection based on the result of the comparison made in step b).

In this case, a severe form of HBV infection is characterized by a decreased amount of HBV immune complexes as set forth elsewhere herein while a mild form of HBV infection is characterized by an increased amount of HBV immune complexes. In this case, the reference amounts are, preferably, those which are the average or mean amounts found in a subjects suffering from HBV prior to the treatment for a given population or cohort of subj ects.

A "mild form" of HBV infection is, preferably, a form which can be treated by interferon treatment as set forth elsewhere herein while a "severe form" is, preferably, a chronic HBV infection which can not be treated by interferon treatment.

The present invention relates also to the use of the amount of HBV immune complexes in a sample from a subject suffering from HBV infection or a detection agent for the HBV immune complexes in such a sample for identifying a subject suffering from HBV infection as being susceptible to interferon treatment.

Suitable detection agents which can be used for determining the HBV complexes present in a sample are described elsewhere herein in detail.

Moreover, the present invention also relates to a device for identifying a subject suffering from hepatitis B virus (HBV) infection as being susceptible to interferon treatment, comprising an analyzing unit for determining the amount of HBV immune complexes and an evaluation unit for comparing said amount to a reference amount and for identifying a subject suffering from HBV infection as being susceptible to interferon treatment.

The term "device" as used herein relates to a system of means comprising at least the aforementioned means operatively linked to each other as to allow the differentiation. Preferred means for determining the amount of the said HBV immune complexes and means for carrying out the comparison are disclosed above in connection with the methods of the invention. How to link the means in an operating manner will depend on the type of means included into the device. For example, where means for automatically determining the amount of the HBV immune complexes are applied, the data obtained by said automatically operating means can be processed by, e.g., a computer program in order to establish a diagnosis (i.e. identifying a subject being susceptible for the interferon treatment). Preferably, the means are comprised by a single device in such a case. Said device may accordingly include an analyzing unit for the measurement of the amount of the HBV immune complexes in a sample and an evaluation unit for processing the resulting data for the diagnosis. Alternatively, where means such as test stripes are used for determining the amount of the HBV immune complexes, the means for diagnosing may comprise control stripes or tables allocating the determined amount to an amount known to be accompanied with response to standard interferon treatment or with non-response to interferon treatment. Preferred means for detection are disclosed in connection with embodiments relating to the methods of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further inventive skills. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonance devices, NMR spectro-meters, mass- spectrometers etc.) or evaluation units/devices referred to above in accordance with the methods of the invention.

The present invention contemplates a kit comprising instructions to carry out the method of the present invention, a detection agent for determining the amount of an HBV immune complex, and, preferably, standards for reference amounts allowing identifying a subject suffering from HBV infection as being susceptible to standard interferon treatment.

The term "kit" as used herein refers to a collection of the aforementioned components, preferably, provided separately or within a single container. The container, also preferably, comprises instructions for carrying out the method of the present invention. Examples for such the components of the kit as well as methods for their use have been given in this specification. The kit, preferably, contains the aforementioned components in a ready-to-use formulation. Preferably, the kit may additionally comprise instructions, e.g., a user's manual for interpreting the results of any determination(s) with respect to the diagnoses provided by the methods of the present invention. Particularly, such manual may include information for allocating the amounts of the determined HBV immune complex to the kind of diagnosis. Details are to be found elsewhere in this specification. Additionally, such user's manual may provide instructions about correctly using the components of the kit for determining the amount(s) of the respective biomarker. A user's manual may be provided in paper or electronic form, e.g., stored on CD or CD ROM. The present invention also relates to the use of said kit in any of the methods according to the present invention.

All references cited in this specification are herewith incorporated by reference with respect to their entire disclosure content and the disclosure content specifically mentioned in this specification.

### FIGURES

**Fig. 1**: Principle of HBV immune complex detection; Anti-HBsAg capture antibodies (Biotin aHBS1, Biotin aHBS2) are bound to a solid surface. HBV immune complexes (comprising HBsAg and anti-HBsAg IgG) are captured to the surface and detected by a monoclonal IgM with a low affinity, but a high avidity to human IgG conjugated to Digoxigenin (anti-<aggregated human IgG> IgM-Digoxigenin.

**Fig. 2**: Schematic drawing of the study design.

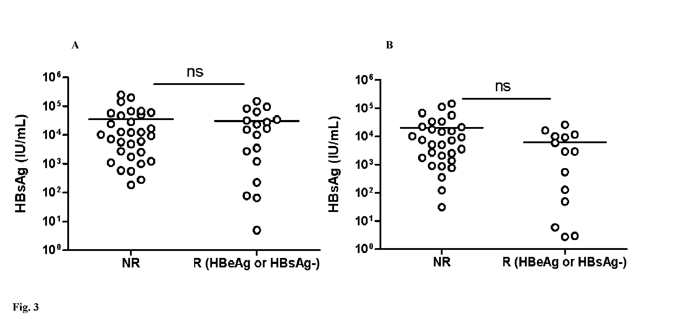

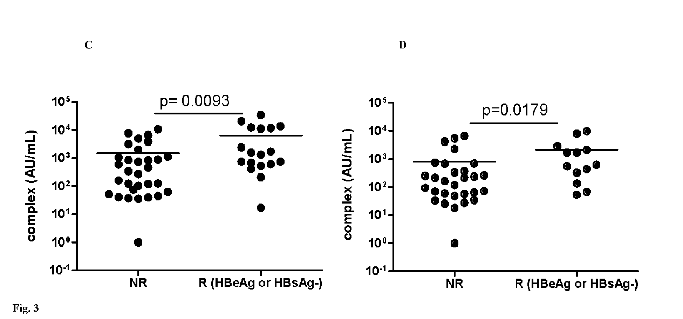

**Fig. 3**: Amount of HBV immune complexes vs. response to standard treatment in a cohort not differentiated according to HBeAg status; A) and B): concentration ofHBsAg at week 0 (i.e. before start of therapy, A) and at week 12 (B) of therapy in serum samples from patients responding (R) or non-responding (NR) to standard therapy. Differences between responders and non-responders are not statistically significant. C) and D): concentration of HBsAg/anti-HBsAg IgG complex at week 0 (C) and at week 12 (D) of therapy in serum samples from patients responding (R) or non-responding (NR) to standard therapy. Differences between responders and non-responders are statistically significant at week 0 (p=0.0093) and at week 12 (P=0.0179).

**Fig. 4**: ROC curves for the data obtained in Fig. 3.

### EXAMPLES

The following Examples shall merely illustrate the invention. They shall not be construed, whatsoever, to limit the scope of the invention.

### Example 1

### Production of monoclonal mouse IgM antibodies with rheumatoid factor-like specificity

### Immunogen: H-IgG polymer:

10 mg human IgG1 (Sigma Company) is dissolved in 0.6 ml 25 mM bicarbonate buffer pH 9.5. After adding 3.5 µl 12.5% glutardialdehyde solution, it is incubated for 2 hours at room temperature. Subsequently it is cooled in an ice bath, adjusted to pH 8.3 with 50 mM triethanolamine solution pH 8.0 and 0.15 ml freshly prepared sodium boron hydride solution (8 mg boron hydride/ml water) is added. After 2.5 hours at 0°C the preparation is dialysed for 16 hours at 4°C against 10 mM potassium phosphate buffer/0.2 M NaCl, pH 7.5. The dialysate containing IgG polymer is stored in aliquots at 80°C or used for immunization and for specificity tests in culture supernatants of hybridoma cells. H-IgG3 polymer is produced in a similar manner starting from human IgG3 (Sigma Company). Immunization of mice:

12 week old, female Balb/c mice are firstly immunized intraperitoneally with 100 µg H-IgG1 or IgG3 polymer together with the adjuvant CFA (complete Freund's adjuvant). After 8 days a further immunization is carried out with 100 µg of the respective IgG polymer in CFA. 13 days after the initial immunization, 200 µg of the respective polymer is administered intraperitoneally without adjuvant, 14 and 15 days after the initial immunization 100 µg was administered in each case intraperitoneally and intravenously. The fusion is carried out after 16 days.

### Production of hybridoma clones:

### Fusion and cloning:

Spleen cells of an immunized mouse are fused with myeloma cells following the method of Galfré, G., Methods in Enzymology 73 (1981) 3-46. Approximately 1 x 10 spleen cells of the immunized mouse are mixed with 2 x 10⁷ myeloma cells (P3X63-Ag8-653, ATCC CRL 1580) and centrifuged (10 min at 300 g and 4°C). The cells are then washed once with RPMI-1640 medium without foetal calf serum (FCS) and again centrifuged at 400 g in a 50 ml conical tube. 1 ml PEG (polyethylene glycol) (molecular weight 4000, Merck, Darmstadt) is added and mixed by pipetting. After 1 min in a water bath at 37°C, 5 ml RPMI 1640 without FCS is added dropwise, mixed, filled up to 50 ml with medium (RPMI 1640 + 10% FCS) and subsequently centrifuged. The sedimented cells are taken up in RPMI 1640 medium containing 10% FCS and sown in hypoxanthine-azaserine selection medium (100 mmol/l hypoxanthine, 1 µg/ml azaserine in RPMI 1640 + 10% FCS). Interleukin 6 (100 U/ml) is added to the medium as a growth factor. After about 10 days the primary cultures were tested for specific antibody synthesis. Primary cultures which show a positive reaction with aggregated human IgG1 but no cross-reaction with monomeric IgG are cloned by means of a fluorescence-activated cell sorter in 96-well cell culture plates. Interleukin 6 (100 U/ml) is added to the medium as a growth additive.

The following hybridoma clones were obtained in this manner:

**Table 1:**

| Monoclonal antibody name | Immunogen | Subclass specificity polymer |
|---|---|---|
| MAb<h-Agg.-IgG>M-3.022.5-IgM | h-IgGl polymer | IgGl>IgG3>IgG4>IgG2 |
| MAb <h-Agg.-IgG>M-1.010.2-IgM | h-IgGl polymer | IgGl>IgG3>IgG4>IgG2 |
| MAb <h-Agg.-IgG>M-1.1.7-IgM | h-IgG3 polymer | IgGl>IgG3>IgG2>IgG4 |

Screening test for monoclonal antibodies having specificity for aggregated human IgG.

Streptavidin-coated microtitre plates (MTPs) are coated with biotinylated human IgG1 or IgG3. Afterwards they are incubated with the monoclonal antibody in the cell culture supernatant. Subsequently the bound antibodies are detected in the usual manner using an anti-<mouse-IgM>-peroxidase (POD) by reaction with a POD substrate.

### Determination of the subclass specificity using human IgG bound to a solid 10 phase:

In order to determine the specificity of the antibodies in the culture supernatant of the hybridoma cells, MTPs coated with recombinant streptavidin (MicroCoat Company, Order No. 12-K 96 N) are coated with 1 µg/ml biotinylated h-IgG (=h-IgG-Bi) of subclass 1 or 2 or 3 or 4 in incubation buffer. Since IgG bound via biotin to a solid phase behaves like aggregated polymeric IgG, this experimental approach can be used to determine the subclass specificity. For this 100 µl h-IgG-Bi solution per well is incubated for 60 minutes at room temperature while shaking and subsequently washed 3 times with 0.9% NaCl / 0.05% Tween ® 20. In the next step 100 µl of the antibody solution to be examined (culture supernatant) is added to a coated well and incubated for 1 hour at room temperature while shaking. After washing 3 times with 0.9% sodium chloride / 0.05% Tween® 20, 100 µl of a POD-labeled Fab fragment of a polyclonal antibody from the goat against mouse IgM (Dianova Company, Order No. 115-036-075, concentration used 0.16 µg/ml incubation buffer) is added in each case to detect bound antibody from the sample, incubated for 1 hour at room temperature while haking and subsequently washed 3 times with 0.9% sodium chloride / 0.05% Tween® 20. Finally 100 µl/well ABTS® substrate (Roche Diagnostics GmbH, Order No. 1684 302) is added and the absorbance at 405/492 nm is measured after 30 min at room temperature in an MR700 Microplate reader from the Dynatech Company.

Incubation buffer: 40 mM Na phosphate, pH 7.4, 200 mM Na tartrate, 0.1% Tween® 20, 0.2% bovine serum albumin.

### Determination of the reactivity / cross-reaction with monomeric, human IgG1:

In order to determine the reactivity / cross-reaction with monomeric, non-aggregated H-IgG1, the monoclonal antibody to be examined is pre-incubated in the test described above with monomeric, non-aggregated IgG1 in increasing concentrations or in excess. If the measured signal remains unchanged at a high level, there is no cross-reaction. If the measured signal decreases, a cross-reaction has occurred.

For this microtitre plates (MTP) (MicroCoat Company, Order No. 12-K 96 N) coated with recombinant streptavidin are coated with 1 µg/ml biotinylated HIgGl (=H-IgG1-Bi) in incubation buffer. 100 µl of the H-IgGl-Bi solution is used per well and incubated for 60 min at room temperature while shaking and subsequently washed 3 times with 0.9% NaCI/ 0.05% Tween® 20. The monoclonal antibody to be tested for cross-reaction is pre-incubated with serial concentrations of up to 1 µg/ml monomeric, non-aggregated IgG1. The pre-incubation takes place in uncoated 96-well MTPs for 1 hour at room temperature while shaking.

In the next step 100 µl of this solution (antibody + non-aggregated, monomeric IgG1 in excess) is added to a coated well and incubated for 1 hour at room temperature while shaking. After washing 3 times with 0.9% sodium chloride / 0.05% Tween® 20, 100 µl of a POD-labeled Fab fragment of a polyclonal antibody from the goat against mouse IgM (Dianova Company, Order No. 115-036-075, concentration used 0.16 µg/ml incubation buffer) is added in each case to detect bound antibody from the sample, incubated for 1 hour at room temperature while shaking and subsequently washed 3 times with 0.9% sodium chloride / 0.05% Tween® 20.

Finally 100 µl/well ABTS® substrate (Roche Diagnostics GmbH, Order No. 1684 302) is added and the absorbance at 405/492 nm is measured after 30 min at room temperature in an MR700 Microplate reader from the Dynatech Company. The monoclonal rheumatoid factor-like binding antibodies that are suitable in the sense of the invention recognize all human IgG subclasses and exhibit less than 10% cross-reaction with monomeric h-IgG in a competition test. If HIgGl polymer is used to determine the reactivity, the measured signal is greatly reduced. Table 1 shows the major properties of the monoclonal antibodies that were found.

### Fermentation of hybridoma clones to isolate monoclonal antibodies:

The hybridoma cells that are obtained are sown at a density of 1x10⁵ cells per ml in RPMI 1640 medium containing 10% FCS and propagated for 7 days in a fermenter (Thermodux Company, Wertheim/_Main, model MCS-104XL, Order No. 144-050). Average concentrations of 100 µg monoclonal antibody per ml are reached in the culture supernatant.

### Isolation of monoclonal MAb <h-Agg.-IgG>M-3.022.5-IgM:

5 mg MAb <h-Agg.-IgG>M-3.022.5-IgM (DSM ACC2873) is adjusted to a total volume of 2 ml with 0.1 M sodium phosphate buffer, pH 8.6. 50 µl of a 1.11 mM solution of digoxigenin-3-O-methyl-carbonyl-ε-aminocaproic acid-N-hydroxysuccinimide ester in dimethyl sulfoxide is added to this solution and subsequently stirred for 60 min at 25°C. The ratio of IgM to activated digoxigenin is 1:10. The IgM-digoxigenin that forms is dialysed against 20 mM potassium phosphate buffer / 0.1 M NaCl / 3% sucrose, pH 7.5. The dialysed IgM-Dig is stored in aliquots at -80°C.

### Example 2

### Fully automated immunoassay on a multi parameter biochip platform (IMPACT)

A multiparameter biochip platform is described in Hornauer, H. et al., BIOspectrum, Special Proteomics 10 (2004) 564-565 and Hornauer, H. et al., Laborwelt 4 (2004) 38-39. To determine complex levels an array-based assay was used (IMPACT - Immunological Multi-Parameter Chip Technology, Roche Diagnostics).

A streptavidin coating is applied over the whole area of a test area of about 2.5 x 6 mm on a black-stained polystyrene support (solid phase). Lines of identical spots of approximately 10 to 20 per line consisting of biotinylated fragments of the therapeutic antibody are applied to the test area in an ink-jet procedure; the diameter per spot is about 150 µm.

The following test-specific reagents were used:
Sample dilution buffer and detection antibody buffer:
   50 mM Tris, pH 6.6; 30 mM MES; 50 mM NaCl; 0.1% detergent (polydocanol); 5mM EDTA; 0.5% Casein; 0.2% preservative (oxypyrion and methylisothiazolone hydrochloride (MIT))

If samples showed very high concentrations of HBV-immunocomplexes and results were not within the measuring range, additional manual sample dilutions were performed using Elecsys Diluent MultiAssay (Ident No. 03609987). Samples were diluted further in 10-fold steps (e.g. 1:10, 1:100, 1:1000)

Wash buffer: 10 mM Tris, 0.01% polydocanol, 0.001% oxypyrion, 0.001% MIT

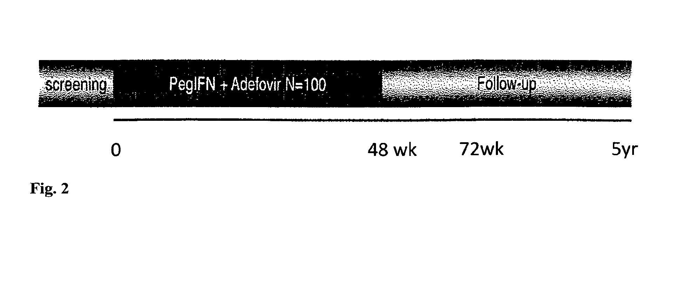

Samples: human sera from chronic HBV patients before and during treatment with Peginterferon alpha-2a (PEGASYS) and Adefovir (Fig. 2, Table 2).

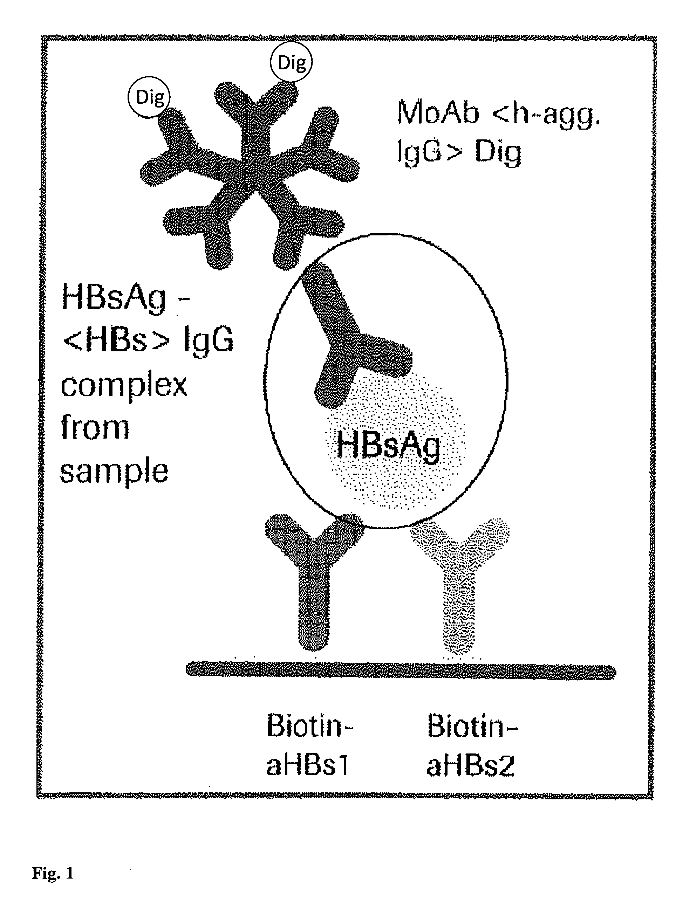

Two different biotinylated antibodies specifically binding to HBs antigen were used as biotinylated capture antibodies. Immune complexes of HBs antigen and anti-HBs antibodies were bound to the solid phase-bound capture antibodies. Immune complexes were detected by a monoclonal IgM antibody specific for human aggregated IgG (MAb<h-Agg.-IgG>M-3.022.5-IgM)(Fig. 1).

The samples were diluted 1:5 with the sample dilution buffer for the measurement. The diluted samples were incubated for 12 min at 37°C. After aspirating the sample and washing the test field with wash buffer, they were incubated with the MAb <h-Agg.-IgG>M-3.022.5-IgM (DSM ACC2873), an antibody labeled with digoxin (Dig-labeled monoclonal antibody <h-Agg.-IgG>), for 6 min at 37°C with a subsequent washing step. After incubation with a fluorescently labeled <Dig> antibody for 3 min at 37°C and subsequently washing and suction drying the test field, the signals were detected by a CCD camera (Fig. 3). The concentration could be calculated with suitable complex-calibrators.

**Table 2: study population**

| HBeAg positive (n=29) | | | HBeAG negative (n=21) | |
|---|---|---|---|---|
| HBsAg seroconversion | HBeAg seroconversion | non responder | HBsAg seroconversion | non-responder |
| n=4 | n=10 | n=15 | n=5 | n=16 |

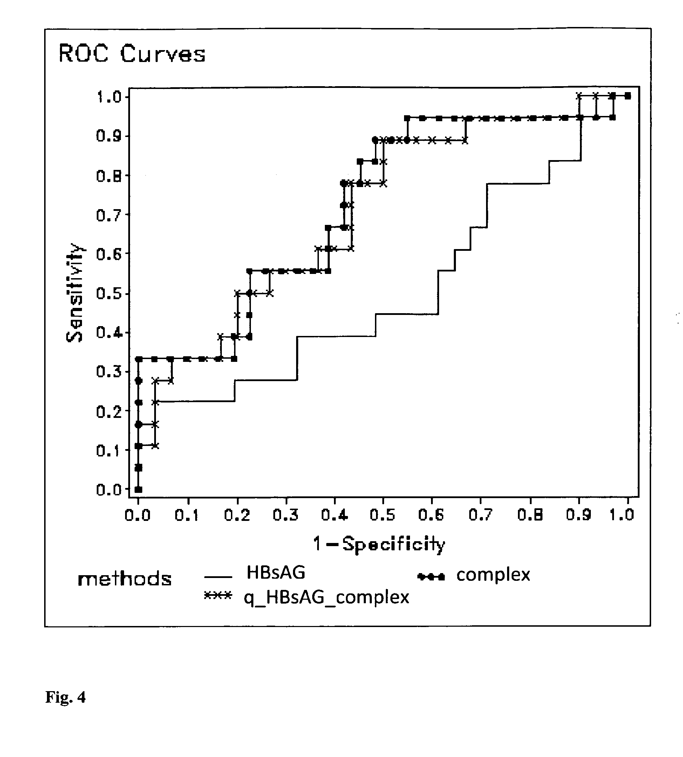

**Table 3: Statistical values for the ROC analysis (Fig. 4)**

| test | auc | std | LCL | UCL |
|---|---|---|---|---|
| complex (HBsAg/antiHBsAG IgG) | 0.7258 | 0.0768 | 0.5753 | 0.8764 |
| HBsAg | 0.5054 | 0.0920 | 0.3250 | 0.6858 |
| ratio HBsAg / complex | 0.7093 | 0.0776 | 0.5572 | 0.8613 |

## Claims

1. A method for identifying a subject suffering from hepatitis B virus (HBV) infection as being susceptible to interferon treatment, said method comprising the steps of:
a) determining, in a sample of said subject, the amount of HBV immune complexes,
b) comparing the amount of HBV immune complexes obtained in step a) to a reference amount, and
c) identifying a subject suffering from HBV infection as being susceptible to interferon treatment based on the result of the comparison made in step b).

2. The method of claim 1, wherein the HBV e antigen status of the subject is unknown.

3. The method of claim 1 or 2, wherein said HBV infection is a chronic HBV infection.

4. The method of any one of claims 1 to 3, wherein the interferon treatment comprises treatment with PEG-interferon and an inhibitor of the viral DNA polymerase.

5. The method of claim 4, wherein said PEG-interferon treatment is PEGasys® and said inhibitor of the viral DNA polymerase is Adefovir®.

6. The method of any one of claims 1 to 5, wherein the HBV immune complexes comprise hepatitis B soluble antigen (HBsAg) and an anti-HBsAG IgG.

7. The method of any one of claims 1 to 6, wherein an increased amount of HBV immune complexes is indicative of a subject being susceptible to interferon treatment while a decreased amount for HBV immune complexes is indicative for a subject being not susceptible to interferon treatment.

8. The method of any one of claims 1 to 7, wherein the sample is a serum sample.

9. The method of any one of claims 1 to 8, wherein the amount of HBV immune complexes is detected with an anti-immunoglobulin antibody.

10. The method of any one of claims 1 to 9, wherein i) the HBV immune complexes comprise anti-HBsAg IgG, anti-HBeAg IgG, or anti-HBcAg IgG and wherein ii) the amount of HBV immune complexes is detected with an anti-(aggregated-humanIgG) IgM.

11. The method of any one of claims 1 to 11, wherein the amount of HBV immune complex is determined with an enzyme-linked immunosorbent assay (ELISA).

12. Use of the amount of HBV immune complexes in a sample from a subject suffering from HBV infection or a detection agent for the HBV immune complexes in such a sample for identifying a subject suffering from HBV infection as being susceptible to interferon treatment.

13. A device for identifying a subject suffering from hepatitis B virus (HBV) infection as being susceptible to interferon treatment, comprising an analyzing unit for determining the amount of HBV immune complexes and an evaluation unit for comparing said amount to a reference amount and for identifying a subject suffering from HBV infection as being susceptible to interferon treatment.

14. A kit comprising instructions to carry out the method of the present invention, a detection agent for determining the amount of an HBV immune complex, and, preferably, standards for reference amounts allowing identifying a subject suffering from HBV infection as being susceptible to standard interferon treatment.

15. The use of claim 12, the device of claim 13, or the kit of claim 14, wherein the HBV e antigen status of the subject is unknown.


SEQUENCE LISTING

<110>  Roche Diagnostics GmbH

<120>  HBV immunocomplexes for response prediction and therapy
       monitoring of chronic HBV patients

<130>  RD30834EP

<160>  3

<170>  PatentIn version 3.5

<210>  1
<211>  400
<212>  PRT
<213>  Hepatitis B virus

<400>  1

Met Gly Gly Trp Ser Ser Lys Pro Arg Gln Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Gly Ala Asn Ser Asn Asn Pro Asp Trp Asp Phe Asn Pro Asn
        35                  40                  45

Lys Asp His Trp Pro Glu Ala Asn Gln Val Gly Ala Gly Ala Phe Gly
    50                  55                  60

Pro Gly Phe Thr Pro Pro His Gly Gly Leu Leu Gly Trp Ser Pro Gln
65                  70                  75                  80

Ala Gln Gly Ile Leu Thr Thr Val Pro Val Ala Pro Pro Pro Ala Ser
                85                  90                  95

Thr Asn Arg Gln Ser Gly Arg Gln Pro Thr Pro Ile Ser Pro Pro Leu
            100                 105                 110

Arg Asp Ser His Pro Gln Ala Met Gln Trp Asn Ser Thr Thr Phe His
        115                 120                 125

Gln Ala Leu Leu Asp Pro Arg Val Arg Gly Leu Tyr Phe Pro Ala Gly
    130                 135                 140

Gly Ser Ser Ser Gly Thr Val Asn Pro Val Pro Thr Thr Ala Ser Pro
145                 150                 155                 160

Ile Ser Ser Ile Phe Ser Arg Thr Gly Asp Pro Ala Pro Asn Met Glu
                165                 170                 175

Ser Thr Thr Ser Gly Phe Leu Gly Pro Leu Leu Val Leu Gln Ala Gly

180                 185                 190

Phe Phe Leu Leu Thr Arg Ile Leu Thr Ile Pro Gln Ser Leu Asp Ser
        195                 200                 205

Trp Trp Thr Ser Leu Asn Phe Leu Gly Gly Ala Pro Thr Cys Pro Gly
    210                 215                 220

Gln Asn Ser Gln Ser Pro Thr Ser Asn His Ser Pro Thr Ser Cys Pro
225                 230                 235                 240

Pro Ile Cys Pro Gly Tyr Arg Trp Met Cys Leu Arg Arg Phe Ile Ile
                245                 250                 255

Phe Leu Phe Ile Leu Leu Leu Cys Leu Ile Phe Leu Leu Val Leu Leu
            260                 265                 270

Asp Tyr Gln Gly Met Leu Pro Val Cys Pro Leu Leu Pro Gly Thr Ser
        275                 280                 285

Thr Thr Ser Thr Gly Pro Cys Lys Thr Cys Thr Ser Pro Ala Gln Gly
    290                 295                 300

Thr Ser Met Phe Pro Ser Cys Cys Cys Thr Lys Pro Ser Asp Gly Asn
305                 310                 315                 320

Cys Thr Cys Ile Pro Ile Pro Ser Ser Trp Ala Phe Ala Arg Phe Leu
                325                 330                 335

Trp Glu Trp Ala Ser Val Arg Phe Ser Trp Leu Ser Leu Leu Val Pro
            340                 345                 350

Phe Val Gln Trp Phe Val Gly Leu Ser Pro Thr Val Trp Leu Ser Val
        355                 360                 365

Ile Trp Met Met Trp Tyr Trp Gly Pro Cys Leu Tyr Asn Ile Leu Ser
    370                 375                 380

Pro Phe Leu Pro Leu Leu Pro Ile Phe Phe Cys Leu Trp Val Tyr Ile
385                 390                 395                 400

<210>  2
<211>  212
<212>  PRT
<213>  Hepatitis B virus

<400>  2

Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Asp Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210>  3
<211>  214
<212>  PRT
<213>  Hepatitis B virus

<400>  3

Met Gln Leu Phe His Leu Cys Leu Ile Ile Ser Cys Thr Cys Pro Thr
1               5                   10                  15

Val Gln Ala Ser Lys Leu Cys Leu Gly Trp Leu Trp Gly Met Asp Ile

            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Asn Asn Leu Glu Asp Pro Ala Ser Arg Asp
            100                 105                 110

Leu Val Val Asn Tyr Val Asn Thr Asn Met Gly Leu Lys Ile Arg Gln
        115                 120                 125

Leu Leu Trp Phe His Ile Ser Cys Leu Thr Phe Gly Arg Glu Thr Val
    130                 135                 140

Leu Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Asp Arg Gly Arg Ser Pro Arg Arg Arg Thr Pro
            180                 185                 190

Ser Pro Arg Arg Arg Arg Ser Gln Ser Pro Arg Arg Arg Arg Ser Gln
        195                 200                 205

Ser Arg Glu Ser Gln Cys
    210